# EUROPÄISCHE PATENTANMELDUNG

(11) **EP 4 714 999 A1**
(43) Veröffentlichungstag der Anmeldung: **25.03.2026**
(21) Anmeldenummer: 25196376.5
(22) Anmeldetag: 18.08.2025
(51) Int. Cl.: C08G 77/12, A61K 8/58, A61K 8/891, C08G 77/38, C08G 77/50, C08K 5/01

(54) **BIOBASIERTE OLEFINE IN SILIKONEN**

(30) Priorität: 19.08.2024 DE 102024123535
(71) Anmelder: CHT Germany GmbH, 72072 Tübingen (DE)
(72) Erfinder: Selig, Alexander, 72072 Tübingen (DE); Klein, Christina, 72072 Tübingen (DE); Bachmann, Julian, 72072 Tübingen (DE); Jürgens, Eva, 72072 Tübingen (DE)
(74) Vertreter: dompatent

(57) **Zusammenfassung**

Die vorliegende Erfindung betrifft ein Verfahren zur Herstellung eines biobasierten alkyl-modifizierten Silikonpolymers sowie seine Verwendung in Hygieneartikel und/oder in kosmetischen Produkten. Der biobasierte Anteil wird erfindungsgemäß durch die Modifizierung durch biobasierte Olefine erreicht.

## Beschreibung

Die vorliegende Erfindung betrifft ein Verfahren zur Herstellung eines biobasierten alkyl-modifizierten Silikonpolymers sowie seine Verwendung in Hygieneartikeln und/oder in kosmetischen Produkten. Der biobasierte Anteil wird erfindungsgemäß durch die Verwendung von biobasierten Olefinen in der Silikonpolymerherstellung erreicht.

Silikone haben eine vielseitige Anwendung. Sie sind wärmebeständig, hydrophob, dielektrisch und gelten in der Regel als physiologisch verträglich, weshalb sie für den Hautschutz, die kosmetische Hautpflege und in der plastischen Chirurgie eingesetzt werden. Im Gegensatz zu Mineralölen oder Pflanzenölen basieren insbesondere Silikonpolymere auf siloxanbasierten Kettenmolekülen als Grundstruktur. Diese zeichnet sich durch die periodisch alternierende Anordnung von Silizium- und Sauerstoffatomen mit der allgemeinen Summenformel [R¹R²SiO]ₙ aus. Am Siliziumatom sind Reste R kovalent gebunden, welche organische Reste sind, aber auch Halogene und/oder Wasserstoffatome sein können. Somit besitzen sie neben dem anorganischen Anteil auch einen organischen Anteil. Allerdings haben diese organomodifizierten Silikone einen eindeutigen Nachteil: Die Modifikation der Restgruppe R erfolgt durch petrochemisch gewonnene Rohstoffe oder ist nur in geringen Maßen aus natürlichen Rohstoffen möglich. Allerdings gibt es die Anforderungen an Konsumgüter, wie kosmetischen Produkten, Haushaltspflege- und Reinigungsmitteln, sowie Autopflege- und Reinigungsprodukten, dass diese den Einsatz von Rohstoffen mit einem hohen natürlichen Anteil aufweisen. Dies wird auch in vielen Labels gefordert und spiegelt die Nachhaltigkeitsstrategie der UN und EU wieder. Insbesondere bei alkyl-modifizierten Silikonpolymeren besteht die Restgruppe aus petrochemisch gewonnenen Rohstoffen, wie beispielsweise Olefinen.

Herkömmliche alkyl-modifizierte Silikone mit einer Alkylkette mit einer Kohlenstoffanzahl von größer gleich 6 bestehen aus petrochemischen Rohstoffen. Andere Produktklassen haben durch ihre Modifikation, wie beispielsweise alkoxylierte Silikone, nur einen geringen Anteil an natürlichen Rohstoffen <50%, oder werden durch Verdünnungen mit Wasser auf einen Anteil >50% eingestellt und verlieren dabei an Qualität.

So offenbart Chieregato et al. wie kurzkettige Olefine aus Biomasse gewonnen werden können (Chemicals and Fuels from Bio-Based Building Blocks, First Edition. Edited by Fabrizio Cavani, Stefania Albonetti, Francesco Basile, and Alessandro Gandini.2016Wiley-VCH Verlag GmbH & Co. KGaA. Published 2016 by Wiley-VCH Verlag GmbH & Co. KGaA, Seite 3-25).

Zurzeit gibt es somit keinen nachhaltigen Ansatz, um diese Silikonpolymere, insbesondere alkyl-modifizierte, mit einem hohen natürlichen Anteil herzustellen.

Es konnte überraschenderweise festgestellt werden, dass der Einsatz von natürlich gewonnen Olefinen in der Synthese von alkyl-modifizierten Silikonpolymeren problemlos möglich ist. Insbesondere konnte festgestellt werden, dass Olefine mit einer Kohlenstoffanzahl von 6 bis 45, besonders 8 bis 18, in einem Verfahren zur Herstellung von biobasierten alkyl-modifizierten Silikonpolymeren eingesetzt werden können. Das Verfahren trägt somit wesentlich zur nachhaltigen Herstellung von alkyl-modifizierten Silikonpolymeren bei.

Es sollte klargestellt werden, dass die Begriffe Silikone, Silikonpolymere, Siloxane und Siloxanpolymere Synonyme sind und die Erfindung somit nicht einschränken.

In einer ersten Ausführungsform wird die zur Erfindung zu Grunde liegende Aufgabe gelöst durch ein Verfahren zur Herstellung eines biobasierten alkyl-modifizierten Silikonpolymers, das zumindest folgende Schritte umfasst:
a) Vorlegen zumindest eines biobasierten endständigen Olefins, wobei das Olefin eine Kohlenstoffanzahl von 6 bis 45 aufweist,
b) Hinzugeben zumindest eines Siloxanpolymers,
c) Hinzugeben zumindest eines Übergangsmetall-Katalysators, welches optional in einem Lösemittel gelöst ist,
d) Umsetzung des Gemisches unter Rühren bei einer Temperatur von 65 °C bis 130 °C, wodurch sich das biobasierte alkyl-modifizierte Silikonpolymer bildet und das erhaltene alkyl-modifizierte Silikonpolymer mindestens einen biobasierten Anteil von mindestens 45 Gew.-% bezogen auf die Masse des Polymers aufweist, weiterhin erfindungsgemäß durch die gewichtsmittlere molare Masse definiert.

Es hat sich überraschenderweise gezeigt, dass durch das erfindungsgemäße Verfahren bio-basierte alkyl-modifizierte Silikonpolymere mit einem sehr hohen Umsatz des Siloxanpolymers hergestellt werden können. Die durch das erfindungsgemäße Verfahren erhaltenen bio-basierten alkyl-modifizierten Silikonpolymere weisen einen hohen biobasierten Anteil auf und werden entsprechend besser vom Endkunden akzeptiert, da die Silikone entsprechend nachhaltiger sind. Das erfindungsgemäße Verfahren zeichnet sich also unter anderem durch eine hohe Energie- und Rohstoffeffizienz aus, wodurch es besonders nachhaltig ist.

Durch die Modifikation eines Silikongerüsts mit Alkylgruppen aus natürlichen Rohstoffen kann ein höherer biobasierter Anteil erreicht werden als in der Literatur beschrieben. Diese Modifikation erfolgt durch ein Olefin mit einer Kohlenstoffkette von C = 6 bis 45.

Durch das Einbringen von Seitenketten mit einem Massenanteil von mindestens 51 % erfüllt die Anforderungen an das Silikonpolymer, sodass es möglich ist dadurch Formulierungen mit einem natürlichen Anteil größer >50 % herzustellen. Somit bietet es eine biobasierte Lösung für die Kosmetikindustrie sowie Hygieneprodukte, die bisher ausschließlich durch petrochemische Mittel erreicht wurden.

Im Sinne der vorliegenden Erfindung versteht man unter nicht modifizierten Siloxanpolymere die allgemeine Formel (R¹)₃Si-[O-Si(R²)(R³)]ₙ-O-Si(R¹)₃, wobei R¹, R² und/oder R³ Alkylgruppen oder Wasserstoffatome sind. Diese sind dem Fachmann geläufig und es ist erfindungsgemäß möglich, alle Siloxanpolymere im Verfahren zu nutzen, welche eine Alkylgruppe und/oder zumindest ein Wasserstoffatom aufweisen.

R¹ kann somit ausgewählt werden aus linearen und verzweigten Alkanen oder Wasserstoffatomen, wobei die Alkane eine Kohlenstoffanzahl von C = 1 bis 50 aufweisen, vorzugsweise eine Kohlenstoffanzahl von C = 1 bis 30 aufweisen, besonders bevorzugt eine Kohlenstoffanzahl von C = 1 bis 10 aufweisen. Ganz besonders bevorzugt ist dabei an den Enden des Siloxanpolymers, also als R¹, Wasserstoff, Methyl, Ethyl, Isopropyl, Propyl, Butyl und/oder Isobutyl, noch bevorzugter wird R¹ ausgewählt aus Methyl oder Wasserstoff.

R² kann mit R¹ identisch sein und kann somit auch ausgewählt werden aus linearen und verzweigten Alkanen oder Wasserstoffatomen, wobei die Alkane eine Kohlenstoffanzahl von C = 1 bis 50 aufweisen, vorzugsweise eine Kohlenstoffanzahl von C = 1 bis 30 aufweisen, besonders bevorzugt eine Kohlenstoffanzahl von C = 1 bis 10 aufweisen. Ganz besonders bevorzugt ist dabei Wasserstoff, Methyl, Ethyl, Isopropyl, Propyl, Butyl und/oder Isobutyl, am meisten bevorzugt Wasserstoff oder Methyl.

Dies gilt auch für R³, also kann auch R³ mit R¹ identisch sein. So kann auch R³ ausgewählt werden aus linearen und verzweigten Alkanen oder Wasserstoffatomen, wobei die Alkane eine Kohlenstoffanzahl von C = 1 bis 50 aufweisen, vorzugsweise eine Kohlenstoffanzahl von C = 1 bis 30 aufweisen, besonders bevorzugt eine Kohlenstoffanzahl von C = 1 bis 10 aufweisen. Ganz besonders bevorzugt ist dabei Methyl, Ethyl, Isopropyl, Propyl, Butyl und/oder Isobutyl, am meisten bevorzugt Wasserstoff oder Methyl.

Dabei dürfen die im erfindungsgemäßen Verfahren genutzten nicht modifizierten Siloxanpolymers nicht so ausgewählt werden, dass R² und R³ identisch sind, wenn eine davon ein Alkan ist. Sie können aber identisch sein, falls R² und/oder R³ Wasserstoffatome sind.

Es ist aber auch möglich, dass R¹, R² und/oder R³ Wasserstoffatome sind, wobei die Gruppen R¹, R² und/oder R³ unterschiedlich sein können.

In einer bevorzugten Ausführungsform des nicht modifizierten Siloxanpolymers, ist R³ ein Wasserstoffatom, sowie R¹ und R² Alkanreste, wie oben ausgeführt mit der allgemeinen Summenformel (R¹)₃Si-[O-Si(R²)(R³)]ₙ-O-Si(R¹)₃, wobei R¹ und R² gleich oder unterschiedlich sind.

Erfindungsgemäß versteht man unter einem biobasierten endständigen Olefin, endständige Olefine, die aus einer natürlichen Quelle gewonnen werden.

Der hier verwendete Begriff endständige Olefin bezieht sich auf α-Olefine, welche mindestens eine endständige, nicht-konjugierte Kohlenstoff-Kohlenstoff-Doppelbindung aufweisen. Dabei umfasst der Begriff lineare α-Olefine und verzweigte α-Olefine. α-Olefine können eine oder mehrere Kohlenstoffdoppelbindungen zusätzlich zur endständigen Doppelbindung enthalten, die auch als Diene dem Fachmann geläufig sind. Bevorzugt handelt es sich allerdings um einfache lineare oder verzweigte α-Olefine, die dadurch gekennzeichnet sind, dass sie nur eine Doppelbindung an der α-Position aufweisen.

Die biobasierten Olefine, welche im erfindungsgemäßen Verfahren eingesetzt werden, können aus natürlichen Ölen wie Rapsöl, Sonnenblumenöl, Avocadoöl oder anderen natürlichen Ölen gewonnen werden. Es ist auch möglich die bio-basierten Olefine aus anderen Naturstoffen, wie beispielsweise Terpenen, zu gewinnen. Es ist auch denkbar die erfindungsgemäß eingesetzten biobasierten Olefinen aus einem Bio-Alkohol über eine Dehydratisierung zu gewinnen. Insbesondere können natürliche gewonnene Alkohole, die über eine Eliminierungsreaktion zu Olefinen umgewandelt werden, als natürliche Quelle dienen. Dabei spielt es keine Rolle wie die erfindungsgemäßen Olefine hergestellt werden, solange diese aus einer natürlichen Quelle gewonnen worden sind. Somit kommen insbesondere Olefine für das erfindungsgemäße Verfahren in Frage, welche aus natürlichen Alkoholen beziehungsweise Bioalkoholen, insbesondere Fettalkoholen, gewonnen werden können. Als natürliche Quelle versteht man erfindungsgemäß, dass die Olefine aus nachwachsenden Rohstoffen und insbesondere nicht aus petrochemischen Quellen gewonnen werden. Biobasierte Olefine bedeuten im Sinne der vorliegenden Erfindung, dass die Olefine einen Anteil von mindestens 70 Gew.-%, vorzugsweise mindestens 80 Gew.-%, ganz besonders bevorzugt mindestens 90 Gew.-%, noch weiter bevorzugt mindestens 95 Gew.-% aus natürlichen Rohstoffen, bezogen auf die Gesamtmasse des jeweiligen Olefins, stammen.

Ferner wird erfindungsgemäß ein Übergangsmetallkatalysator im Verfahren genutzt, welches optional in einem Lösemittel gelöst ist. Unter einem erfindungsgemäßem Übergangsmetallkatalysator kommen alle Hydrosilylierungskatalysatoren in Frage. Der Fachmann weiß diese entsprechend zu wählen. Besonders bevorzugt werden die Metalle des erfindungsgemäßen Übergansmetallkatalysators ausgewählt aus der Gruppe Platin, Iridium, Rhodium, Ruthenium und/oder Eisen, bevorzugt ausgewählt aus einem Platinkomplex, ganz besonders bevorzugt ausgewählt aus Platin(0)-1,3-divinyl-1,1,3,3-tetramethyldisiloxan-Komplex -Lösungen. Als weitere Alternativen können Katalysatoren auf Basis von Di-m-chloro-bis(1,2-η)-cyclohexen Platin(II)chlorid, Dichloro(1,5-cyclooctadien)platinum, cis-Bis-(benzonitril)-dichloroplatin sowie Hexachloridoplatinsäure betrachtet werden. Erfindungsgemäß kann im Verfahren der Katalysator auch in einem Lösemittel eingesetzt werden. Das Lösemittel ist ausgewählt aus bekannten Polymerisationskatalysatorlösemitteln, wie etwa 1,3-Divinyltetramethyldisiloxan ("Karstedt-Lösung"), Propanol, Butyldiglykol und/oder Ethylen, Allylpolyether oder Allylalkoholen. Es ist ebenfalls aber auch möglich, die Reaktion unter Einsatz lösemittelfreier Katalysatoren durchzuführen.

Durch das erfindungsgemäße Verfahren ist somit ein biobasiertes alkyl-modifiziertes Silikonpolymer erhältlich, welches dadurch gekennzeichnet ist, dass mindestens ein biobasierter Anteil von 50 Gew.-% bezogen auf die Masse des Polymers aufweist. Somit ist es möglich, Silikone mit einem hohen biobasierten Anteil für die Kosmetikindustrie beziehungsweise Hygieneartikelindustrie bereitzustellen, welche zuvor nur zu petrochemisch gewonnen Silikonen Zugang hatten.

Nachfolgend werden bevorzugte Ausführungsformen des erfindungsgemäßen Verfahrens zur Herstellung biobasierter alkyl-modifizierter Silikonpolymere und deren Verwendung ausgeführt, wobei alle Merkmale in beliebiger Art und Weise miteinander kombiniert werden können und das erfindungsgemäße Verfahren und die Verwendung in keiner Weise einschränken.

In einer bevorzugten Ausführungsform kann das Verfahren dadurch gekennzeichnet sein, dass das zumindest eine nicht-modifizierte Siloxanpolymer mindestens eine Einheit der Form -[Si(CH₃)HO]ₙ- mit n = 1 bis 100, bevorzugt n = 25 bis 90, meist bevorzugt n = 50 bis 80 umfassen. Somit kann in dieser bevorzugten Ausführungsform das nicht-modifizierte Siloxanpolymer mit der allgemeinen Summenformel (R¹)₃Si-[O-Si(R²)(R³)]ₙ-O-Si(R¹)³ auch als (R¹)₃Si-[O-Si(CH₃)(H)]₀₋₁₀₀-O-Si(R¹)₃ beschrieben werden, wobei R²= CH₃ und R³= H ist. Ferner kann in dieser bevorzugten Ausführungsform R¹= CH₃, C₂H₅ oder C³H⁷ sein.

Zusätzlich kann in dieser bevorzugten Ausführungsform das zumindest eine nicht-modifizierte Siloxanpolymer eine weitere Einheit der Form -[Si(CH₃)₂O]ₘ-mit m = 1 bis 300, bevorzugt m = 20 bis 200, meist bevorzugt m = 50 bis 150 umfassen. Somit kann in dieser bevorzugten Ausführungsform das nicht-modifizierte Siloxanpolymer mit der allgemeinen Summenformel (R¹)₃Si-[O-Si(R²)(R³)]ₙ-O-Si(R¹)₃ auch als (R¹)₃Si-[O-Si(CH₃)(H)]₀₋₁₀₀-[OSi(CH₃)₂]₁₋₃₀₀-O-Si(R¹)₃ beschrieben werden. Ferner kann in dieser bevorzugten Ausführungsform R¹ = CH₃, C₂H₅ oder C₃H₇, noch bevorzugter R¹ = CH₃ oder H sein.

In einer weiteren bevorzugten Ausführungsform kann im Verfahren das biobasierte endständige Olefin eine Kohlenstoffanzahl mit C = 6 bis 45, bevorzugt C= 8 bis 30, meist bevorzugt C = 9 bis 25, noch bevorzugter C = 10 bis 20, weiterhin bevorzugter C = 11 bis 18 aufweisen. Dabei können die Kohlenstoffe sowohl linear beziehungsweise verzweigt auftreten. Bevorzugterweise werden nur lineare Olefine eingesetzt. Es ist erfindungsgemäß so, dass das Olefin ein endständiges Olefin ist. Damit meint man, dass die Doppelbindung am α-Kohlenstoffatom ist, wobei auch weitere Doppelbindungen im Olefin möglich sind. In einer weiterhin bevorzugten Ausführungsform weist das biobasierte endständige Olefin, nur diese eine endständige Doppelbindung und/oder eine Kohlenstoffanzahl mit C = 6 bis 45, bevorzugt C= 8 bis 30, meist bevorzugt C = 9 bis 25, noch bevorzugter C = 10 bis 20, weiterhin bevorzugter C = 11 bis 18 auf.

In einer weiteren bevorzugten Ausführungsform ist das biobasierte endständige Olefin dadurch gekennzeichnet, dass es zumindest eine Ester-, eine Carbonsäure- und/oder eine Hydroxyl-Gruppe aufweist. Das bedeutet das ein erfindungsgemäßes biobasiertes Olefin entweder drei dieser Gruppen, zwei von drei dieser Gruppen oder nur eine dieser Gruppen aufweist. Dies wird im Folgenden näher erläutert:
Falls das biobasierte endständige Olefin nur eine dieser Gruppen aufweist, so ist diese Gruppe bevorzugt am anderen Ende bezogen auf die endständige Doppelbindung des Olefins positioniert, sodass das Olefin in der Form R⁴-(CH₂)ₙ-CHCH₂ vorliegt, wobei n ausgewählt ist aus n = 6 bis 45, bevorzugt n = 8 bis 30, meist bevorzugt n = 9 bis 25, noch bevorzugter n = 10 bis 20, weiterhin bevorzugter C = 11 bis 18 und R⁴ die funktionelle Gruppe, ausgewählt aus Ester-, Carbonsäure-, oder Hydroxyl-Gruppe ist.

Falls das biobasierte endständige Olefin zwei dieser Gruppen aufweist, so ist einer dieser Gruppen bevorzugt am anderen Ende bezogen auf die endständige Doppelbindung des Olefins positioniert und eine in der Alkyl-Kette, sodass das Olefin in der Form R⁴-(CH₂)ₘ-(CHR⁵)-(CH₂)ₒ-CHCH₂ vorliegt, wobei
m und o aus natürlichen Zahlen so ausgewählt werden, dass ihre Summe eine natürliche Zahl n wie oben definiert ist, und
R⁴ und R⁵ eine Ester-, Carbonsäure-, oder Hydroxyl-Gruppe ist. Dabei können R⁴ und R⁵ gleich oder unterschiedlich sein.

Falls das biobasierten endständige Olefin drei dieser Gruppen aufweist, so ist einer dieser Gruppen bevorzugt am anderen Ende bezogen auf die endständige Doppelbindung des Olefins positioniert und zwei in der Alkyl-Kette, sodass das Olefin in der Form R⁴-(CH₂)ₘ-(CHR⁵)-(CH₂)ₒ-(CHR⁶)- (CH₂)ₚ-CHCH₂ vorliegt, wobei
m, o und p aus natürlichen Zahlen so ausgewählt werden, dass ihre Summe eine natürliche Zahl n wie oben definiert ist, und
R⁴, R⁵ und R⁶ eine Ester-, Carbonsäure-, oder Hydroxyl-Gruppe ist. Dabei können R⁴, R⁵ und R⁶ gleich oder unterschiedlich sein.

Die Estergruppe weist diese die Form von R^{(4/5/6)} = R⁷-OC-O- auf wobei R⁷ ausgewählt wird aus Methyl, Ethyl, Isopropyl, Propyl, Butyl, oder Isobutyl.

In all diesen Ausführungsformen weist die Carbonsäuregruppe die Form R^{(1/2/3)}= HOOC- und die Hydroxyl-Gruppe die Form R^{(1/2/3)}= HO- auf.

In einer weiteren bevorzugten Ausführungsform ist das biobasierte endständige Olefin dadurch gekennzeichnet, dass es keine weiteren funktionellen Gruppen aufweist. Unter funktionellen Gruppen versteht man dabei Halogene, Carbonsäuren, Esther, Ether, Hydroxylgruppen, und andere dem Fachmann geläufige funktionelle Gruppen. Hierbei ist darunter zu verstehen, dass entweder das Olefin die Form (CH₂)ₙ-CHCH₂ oder eine der oben gezeigten Formen haben kann. Das bedeutet, dass das erfindungsgemäß biobasierte endständige Olefin ein "reines" endständiges Olefin der Form (CH₂)ₙ-CHCH₂ ist oder R⁴-(CH₂)ₙ-CHCH₂, R⁴-(CH₂)ₘ-(CHR⁵)-(CH₂)ₒ-CHCH₂, oder auch R⁴-(CH₂)ₘ-(CHR⁵)-(CH₂)ₒ-(CHR⁶)-(CH₂)ₚ-CHCH₂, wobei diese offenbarten Formen des Olefins keine zusätzlichen Gruppen aufweisen. Somit sind alle bevorzugten Ausführungsformen offenbart und stehen nicht im Widerspruch zueinander.

Wie bereits beschrieben, wird das biobasierte endständige Olefin aus einer natürlichen Quelle beziehungsweise natürlichen Rohstoffen gewonnen. Bevorzugt werden dabei natürliche Fettalkohole zur Synthese des Olefins genutzt. Die natürlichen Fettalkohole können dabei aus den natürlichen Quellen, wie beispielsweise Bienenwachs direkt gewonnen und durch eine Eliminierungsreaktion zum entsprechenden Olefin umgewandelt werden. Es ist auch möglich die Fettalkohole aus Fettsäuren aus natürlichen Ölen zu gewinnen. Dabei kann die Fettsäure zum entsprechenden Alkohol reduziert und anschließend eliminiert werden, sodass das einzusetzende Olefin erhalten wird.

In einer bevorzugten Ausführungsform gemäß dem erfindungsgemäßen Verfahren entspricht der biobasierte Anteil des erhaltenen biobasierten alkyl-modifizierten Silikonpolymers einem Anteil von 60 Gew.-% bis 98 Gew.-%, bevorzugt 80 Gew.-% bis 95 Gew.-% bezogen auf die Masse des Polymers. Biobasiert bedeutet dabei im Sinne der vorliegenden Erfindung, dass der Anteil aus einer natürlichen Quelle gewonnen wurde, wie bereits oben beschrieben. Der biobasierte Anteil des finalen Siloxanpolymer wird erreicht, indem die biobasierten endständigen Olefine in das Silikonpolymer eingefügt werden und so ein biobasiertes alkyl-modifiziertes Silikonpolymer ergeben. So kann der biobasierte Anteil dadurch gesteuert werden, dass die Funktionalität der nicht modifizierten Siloxanpolymere geändert wird. Ein erhöhter Anteil an endständigen biobasieren Olefinen gehen mit einer Erhöhung des biobasierten Anteils im Endprodukt einher.

Die Struktur des erfindungsgemäß erhaltenen biobasierten alkyl-modifizierten Silikonpolymers kann somit beschrieben werden, dass die Reste in (R¹)₃Si-[O-Si(R²)(R³)]ₙ-O-Si(R¹)₃ des eingesetzten nicht-modifizierten Siloxanpolymers, R² und/oder R³, durch das biobasierte endständige Olefin substituiert werden, falls die substituierten Reste R² und/oder R³ Wasserstoff sind. Dabei findet eine Hydrosilylierung statt, wobei unter Katalysatoreinfluss ein Silan an die Doppelbindung des α-Kohlenstoffatom eines biobasierten Olefins addiert wird.

In einer weiteren bevorzugten Ausführungsform wird das Verfahren in Schritt d) bei einer Temperatur von 70 °C bis 120 °C, besonders bevorzugt von 75 °C bis 115 °C, ganz besonders bevorzugt von 80 °C bis 110 °C durchgeführt.

In einer weiteren bevorzugten Ausführungsform ist das Verfahren dadurch gekennzeichnet, dass das Siloxanpolymer fast vollständig umgesetzt wird. Unter fast vollständigem Umsatz versteht man in dieser bevorzugten Ausführungsform, dass die freien Wasserstoffe des Siloxanpolymers, also Silane der Form Si-H, umgesetzt werden und folglich diese Gruppen mit dem biobasierten Olefin eine erfindungsgemäße Reaktion eingegangen sind. Dieser Umsatz kann durch den Gehalt der SiH-Gruppen festgestellt werden. In dieser bevorzugten Ausführungsform entspricht ein vollständiger Umsatz einem Umsatz von mindestens 85 %, bevorzugt mindestens 90 %, noch weiter bevorzugt mindestens 95 % oder meist bevorzugt einem Umsatz von mindestens 99 % bezogen auf die Stoffmenge des eingesetzten Siloxanpolymers.

In einer weiteren bevorzugten Ausführungsform ist das Verfahren dadurch gekennzeichnet, dass die erfindungsgemäße Reihenfolge der Schritte a), b), c) und d) so durchgeführt werden. Dabei kann aber a) und b) vertauscht werden, solange die vorgelegte Komponente die Reaktionstemperatur aufweist. Dadurch wird ein effizientes Verfahren mit einem hohen Umsatz gewährleistet. Es sollte hier noch klargestellt werden, dass andere Verfahrensschritte zwischen den Schritten eingebaut werden können und diese nicht ausgeschlossen sind.

Ferner wird erfindungsgemäß auch die Verwendung eines biobasierten alkyl-modifizierten Silikonpolymers, welches bevorzugt durch ein Verfahren gemäß der vorliegenden Erfindung erhalten wurde, in kosmetischen Produkten oder Hygieneprodukten als Hilfsstoff offenbart. Nachfolgend werden mögliche erfindungsgemäße Verwendungen näher erläutert:

Erfindungsgemäß erhaltene biobasierte alkyl-modifizierte Silikonpolymere können als Emollient in kosmetischen und Personal Care Produkten verwendet werden. Durch die Modifikation lässt sich je nach Alkyl-Kettenverteilung das Hautgefühl kontrollieren. Zusätzlich biete die Produktklasse zusätzliche Effekte wie z.B. Kontrolle der Textur, verbessertes Spreitverhalten und Verteilbarkeit, geringere Klebrigkeit, Verbesserung der Wasserabweisung, Pigmentdispergierung, filmbildende Eigenschaften und Lösungsvermittler, sodass eine Vielzahl von Verwendungen möglich ist. Die Produkte eignen sich auch hervorragend für den Einsatz in Polituren oder Wachsformulierungen zur Pflege von harten Oberflächen und Leder. Durch die Modifikation können hier eine besondere Haptik und die nötige Wasserabweisung sowie Glanzeffekte erzielt werden.

Eine Kombination aus amino- oder quaternären Stickstoffgruppen und erfindungsgemäß erhaltene biobasierte alkyl-modifizierte Silikonpolymere können als Konditionierungsmittel und Emulgatoren in Haarpflege und Reinigungsprodukte zum Einsatz kommen und die Kämmbarkeit, die Glätte sowie die Haptik verbessern. Zudem erzielen die Produkte durch das erfindungsgemäß biobasierte erhaltene alkyl-modifizierte Silikonpolymer einen Farb- und Hitzeschutz. Bei der Verwendung in Reinigungs- und Pflegeprodukte für Haushalt-, Wäsche- und Automobilanwendungen erzielen die Produkte einen Glanzeffekt, Wasserabweisung und können die Haptik verbessern.

Eine Kombination aus Amido-Amino-Gruppen (Reaktion aus Amino-Gruppen und Glucon-Delta-Lacton) und erfindungsgemäß biobasierte erhaltene alkyl-modifizierte Silikonpolymer können als PEG-freier Silikonemulgator für W/O, O/W, Si/W und W/Si in Kosmetischen und Personal Care Produkten verwendet werden. Zudem bieten diese Produkte neben der Emulgatorfunktion noch zusätzliche Effekte wie z.B. Pigmentdispergierung, Verbesserung der Textur oder Reduzierung der Klebrigkeit. Des Weiteren eigenen sich diese Art von Polymeren als Konditionierungsmittel in Haarpflege- und Reinigungsprodukten. Die Modifikation zeichnet sich durch eine besondere Haptik und Verbesserung der Trockenkämmbarkeit aus. Als weiteres Anwendungsgebiet sind Polituren, Weichspüler und Reinigungsmittel zu nennen.

Die mäßige Hydrophobie der Polymere eignet sich besonders gut für Weichspüler und Bodenpflege-/Reinigungsprodukte, da in der ersteren Hydrophilie benötigt wird und im zweiten Fall nur Nässeschutz. Zudem können die Produkte die Intensität von Duftstoffen verbessern und sorgen für eine längere Frische.

Eine Kombination aus Polyether oder Polyglykolen und erfindungsgemäß erhaltene biobasierte alkyl-modifizierte Silikonpolymere können als Silikonemulgator/Tensid für W/O, O/W, Si/W und W/Si in Kosmetischen und Personal Care Produkte verwendet werden, zudem bieten diese Produkte neben der Emulgatorfunktion noch zusätzliche Effekte wie zum Beispiel Pigmentdispergierung, Verbesserung der Textur, Reduzierung der Klebrigkeit und können als mildes Tensid die Reinigungswirkung unterstützen.

Das Konzept der vorliegenden Erfindung wird mit den nachfolgenden Ausführungsbeispielen näher erläutert und schränken den Gegenstand der Erfindung nicht auf diese ein.

Dem Fachmann bekannt wird die Menge an reaktiven SiH Funktionalitäten des eingesetzten Polysiloxans in mol/kg angegeben. Der Allylgehalt des eingesetzten Olefins wird über eine theoretisch gerechnete Iodzahl (IZ) charakterisiert.

Dem Fachmann weiterhin geläufig wird der Reaktionsumsatz über die gasvolumetrische Verdrängung einer gesättigten wässrigen Kaliumchloridlösung bei Kontakt des Polysiloxans mit einer butanolischen 10%igen (bezogen auf den Massenanteil des Salzes) Kaliumhydroxidlösung bestimmt.

Alle erhaltenen polymeren Materialien wurden über die gewichtsmittlere (*M*_{w}) molare Masse mittels Gel-Permeations-Chromatographie charakterisiert (Infinity 1260, Agilent).

So erhaltene, bei 22 °C feste Materialien wurden über den Schmelzpunkt mittels dynamischer Differenz-Thermoanalyse (DIN EN ISO 11357-1:2016, EN ISO 11357-2:2020, EN ISO 11357-3:2018) charakterisiert (DSC 204 F1 PH ASC Phoenix^{®}, Netzsch Gerätebau GmbH, zusätzlich ausgestattet mit einem Intracooler der Fa. Huber, *T*ₛₜₐᵣₜ = -75 °C, *T*_{ende} = 200 °C, *Δ*T = 10 K min⁻¹).

So erhaltene, bei 22 °C flüssige Materialien wurden zusätzlich zur Schmelzpunktbestimmung über die kinematische Viskosität mittels Rotationsviskosimetrie (60 rpm, 22 °C, Aufsatz L2, HAAKE^{™} Viscotester^{™} C, Thermo Scientific^{™}) charakterisiert.

### Herstellung des biobasierten alkyl-modifizierten Silikonpolymers:

### Beispiel 1: Umsetzung eines biobasierten C10-C18 endständigen Olefins (IZ=113)

In einem 250 mL Reaktionsgefäß wurden 140 g des C10-C18 Olefins vorgelegt und unter Rühren auf 100 °C temperiert. Nach Erreichen der geforderten Temperatur wurde unter stetigem Rühren 30,0 g eines Wasserstoffsiloxans der mittleren Formel:

(CH₃)₃Si-[O-Si(CH₃)(H)]₆₀₋₈₀-O-Si(CH₃)₃,

charakterisiert durch einen Wasserstoffgehalt von 16,1 mol/kg sowie eines berechneten Molekulargewichts von 3800-5000 Da, innerhalb 75 Minuten zudosiert sowie mit 0,0453 g einer verdünnten Karstedt-Lösung in Dowanol DPM^{®} (Dipropylen glycol methylether, Dow Chemical, USA) so versetzt, dass ein Pt-Gehalt von 8 ppm in der Reaktionsmischung vorliegt. Der Reaktionsfortschritt wurde über die gasvolumetrische SiH Bestimmung verfolgt. Nach 12 h wurde ein SiH-Gehalt von <90 ppm ermittelt und die Reaktion damit als abgeschlossen definiert. Dies entspricht einem Umsatz von 99,4 % des Siloxanpolymers. Das so erhaltene Produkt ist bei 22 °C fest bis wachsartig. Es wurde ein Schmelzpunkt von 34 °C gemessen und eine gewichtsmittlere Molare Masse von *M*_{w} = 28000 Da ermittelt.

### Beispiel 2: Umsetzung eines biobasierten C10-C18 endständigen Olefins (IZ=113)

In einem 250 mL Reaktionsgefäß wurden 127 g des C10-C18 Olefins vorgelegt und unter Rühren auf 80 °C temperiert. Nach Erreichen der geforderten Temperatur wurde unter stetigem Rühren 70,0 g eines Wasserstoffsiloxans der mittleren Formel:

(CH₃)₃Si-[O-Si(CH₃)₁₅₋₃₀(H)]₁₅₋₃₀-O-Si(CH₃)₃,

charakterisiert durch eine Valenz von 7,04 mol/kg sowie eines berechneten Molekulargewichts von 2200-4200 Da, innerhalb 60 Minuten zudosiert sowie mit 0,0504 g einer Karstedt-Lösung im Lösemittel Dowanol DPM^{®} (Dow Chemical, USA) so versetzt, dass ein Pt-Gehalt von 8 ppm in der Reaktionsmischung vorliegt. Nach vollständiger Zudosierung des Wasserstoffsiloxans wurde die Temperatur auf 110 °C erhöht. Der Reaktionsfortschritt wurde über die gasvolumetrische SiH Bestimmung verfolgt. Nach 5 h wurde ein SiH-Gehalt von <25 ppm ermittelt und die Reaktion damit als abgeschlossen definiert. Dies entspricht einem Umsatz von 99,6 % des Siloxanpolymers. Das so erhaltene Produkt ist bei 22 °C flüssig sowie klar bis gelblich. Es wurde eine dynamische Viskosität von 143 mPas sowie einen Schmelzpunkt von 13,9 °C gemessen und eine gewichtsmittlere Molare Masse von *M*_{w} = 11600 Da ermittelt.

### Beispiel 3: Umsetzung eines biobasierten endständigen Olefins mit Esterfunktionalisierung (IZ=138)

In einem 250 mL Reaktionsgefäß wurden 153 g des Olefins vorgelegt und unter Rühren auf 100 °C temperiert. Nach Erreichen der geforderten Temperatur wurde unter stetigem Rühren 40 g eines Wasserstoffsiloxans der mittleren Formel:

(CH₃)₃Si-[O-Si(CH₃)(H)]₆₀₋₈₀-O-Si(CH₃)₃,

charakterisiert durch eine Valenz von 16,1 mol/kg sowie eines berechneten Molekulargewichts von 3800-5000 Da, innerhalb 60 Minuten zudosiert sowie mit 0,0515 g einer Karstedt-Lösung im Lösemittel Dowanol DPM^{®} (Dow Chemical, USA) so versetzt, dass ein Pt-Gehalt von 8 ppm in der Reaktionsmischung vorliegt. Der Reaktionsfortschritt wurde über die gasvolumetrische SiH Bestimmung verfolgt. Nach 5 h wurde ein SiH-Gehalt von <70 ppm ermittelt und die Reaktion damit als abgeschlossen definiert. Dies entspricht einem Umsatz von 99,6 % des Siloxanpolymers. Das so erhaltene Produkt ist bei 22 °C flüssig sowie klar bis gelblich. Es wurde eine dynamische Viskosität von 385 mPas sowie einen Schmelzpunkt von -22 °C gemessen und eine gewichtsmittlere Molare Masse von *M*_{w} = 11200 Da ermittelt.

### Beispiel 4: Umsetzung eines biobasierten C8-C14 endständigen Olefins (IZ=181)

In einem 250 mL Reaktionsgefäß wurden 114 g des Olefins vorgelegt und unter Rühren auf 80 °C temperiert. Nach Erreichen der geforderten Temperatur wurde unter stetigem Rühren 100 g eines Wasserstoffsiloxans der mittleren Formel:

(CH₃)₃S-[O-Si(CH₃)₁₅₋₃₀(H)]₁₅₋₃₀-O-Si(CH₃)₃,

charakterisiert durch eine Valenz von 7,04 mol/kg sowie eines berechneten Molekulargewichts von 2200-4200 Da, innerhalb 60 Minuten zudosiert sowie mit 0,0568 g einer Karstedt-Lösung im Lösemittel Dowanol DPM^{®} (Dow Chemical, USA) so versetzt, dass ein Pt-Gehalt von 8 ppm in der Reaktionsmischung vorliegt. Nach vollständiger Zudosierung des Wasserstoffsiloxans wurde die Temperatur auf 110 °C erhöht. Der Reaktionsfortschritt wurde über die gasvolumetrische SiH Bestimmung verfolgt. Nach 6 h wurde ein SiH-Gehalt von <20 ppm ermittelt und die Reaktion damit als abgeschlossen definiert. Dies entspricht einem Umsatz von 99,6 % des Siloxanpolymers. Das so erhaltene Produkt ist bei 22 °C flüssig sowie klar sowie gelblich. Es wurde eine dynamische Viskosität von 66 mPas sowie einen Schmelzpunkt von -57 °C gemessen und eine gewichtsmittlere molare Masse von *M*_{w} = 7900 Da ermittelt.

### Formulierung einer feuchtigkeitsspendenden Tagescreme: B

| Phase | Inhaltsstoff | Biobasierter Ursprung in Vol.-% | Petrochemischer Ursprung in Vol.-% |
|---|---|---|---|
| A | Isopropylmyristat | 2,00 | 2,00 |
| | Mandelöl | 0,85 | 0,85 |
| | Silikonpolymer | 6,00 | - |
| | Silikonpolymer | - | 6,00 |
| | Caprylic/Capric Triglyceride | 11,00 | 11,00 |
| | Cetearly-glucoside | 2,00 | 2,00 |
| | Tocopherol acetate | 0,25 | 0,25 |
| | Fragrance | 0,10 | 0,10 |
| B | Glycerin | 10,00 | 10,00 |
| | Trinatriumcitrat | 0,50 | 0,50 |
| | Natriumbenzoat | 0,30 | 0,30 |
| | Wasser | Restmenge bis 100 | Restmenge bis 100 |
| | Menge an petrochemischen Rohprodukten* | <1 | 5,4 |
| | Gehalt an natürlichem Ursprung der der Formulierung gemäß ISO 16128-1** | > 99,3 | >93,7 |

| | | | |
|---|---|---|---|
| * Angaben in Vol.% ** prozentuale Angabe | | | |

Herstellung:
Die Bestandteile der Phase A wurden gemeinsam in einem Behältnis auf 65 °C erwärmt und miteinander verrührt. Die Bestandteile der Phase B wurden gemeinsam in einem Behältnis auf 85 °C erwärmt und miteinander verrührt. Anschließend wurde Phase B in Phase A einemulgiert und anschießend unter Rühren auf 20 °C abgekühlt.

### Berechnung für die Einstufung nach ISO 16128-1:

Silikonpolymer gemäß Beispiel 1 modifiziert mit dem biobasierten endständigen Olefin C = 16:

| | In MW | Angabe in Gew.-% |
|---|---|---|
| Fossiler Anteil | 1154 | 6 |
| Mineralischer Anteil | 138 | 1 |
| Bioanteil | 17100 | 93 |
| Gesamt | 18392 | 100 |

Somit ergab sich für das erfindungsgemäße Silikonpolymer ein Bioanteil von 93%, wohingegen petrochemischen Silikonpolymere keinen Bioanteil aufweisen.

### Formulierung eines Conditioner für Hair Care: C

| Phase | Inhaltsstoff | Biobasierter Ursprung in Vol.-% | Petrochemischer Ursprung in Vol.-% |
|---|---|---|---|
| A | Wasser | Restmenge auf 100,00 | Restmenge auf 100,00 |
| B | Cetearylalcohol | 5,00 | 5,00 |
| | Behentrimonium chlorid 85% | 3,00 | 3,00 |
| | Cetylester | 1,00 | 1,00 |
| | Jojobaöl | 0,50 | 0,50 |
| | Kokosnussöl | 0,50 | 0,50 |
| | Silikonpolymer | 2,50 | - |
| | Silikonpolymer | - | 2,50 |
| C | Na-Benzoate und K-Sorbate | 0,75 | 0,75 |
| | Milchsäure 80% | Auf pH 3,7 - 4,5 | Auf pH 3,7 - 4,5 |
| | Tocopherol acetate | 0,15 | 0,15 |
| | Fragrance | 0,10 | 0,10 |
| | Menge an petrochemischen Rohprodukten* | <3 | <3 |
| | Gehalt an natürlichem Ursprung der der Formulierung gemäß ISO 16128-1** | >99 | >97 |

| | | | |
|---|---|---|---|
| * Angaben in Vol.% ** prozentuale Angabe und mineralischen Ursprung berücksichtigt | | | |

Herstellung:
Die Bestandteile der Phase B wurden gemeinsam in einem Behältnis auf 85 °C erwärmt und miteinander verrührt. Die Phase A wurde auf 85 °C erwärmt. Anschließend wurde Phase B in Phase A einemulgiert und anschießend unter Rühren auf 40 °C abgekühlt und die Inhaltsstoffe der Phase C hinzugegeben sowie der pH-Wert eingestellt mittels Milchsäure.

### Berechnung für die Einstufung nach ISO 16128-1:

Silikonpolymer gemäß Beispiel 2 modifiziert mit dem biobasierten endständigen Olefin C = 14-16:

| | In MW | Angabe in Gew.-% |
|---|---|---|
| Fossiler Anteil | 930 | 13 |
| Mineralischer Anteil | 1744 | 24 |
| Bioanteil | 4500 | 63 |
| Gesamt | 7174 | 100 |

Somit ergab sich für das erfindungsgemäße Silikonpolymer ein Bioanteil von 63 %, wohingegen petrochemischen Silikonpolymere keinen Bioanteil aufweisen.

Es hat sich herausgestellt, dass die erfindungsgemäßen biobasierten Silikonpolymere problemlos eingesetzt werden können. Dadurch können sowohl Richtlinien eingehalten als auch die Akzeptanz bei den Verbrauchern erhöht werden.

## Patentansprüche

1. Verfahren zur Herstellung eines biobasierten alkyl-modifizierten Silikonpolymers, das zumindest folgende Schritte umfasst:
a) Vorlegen zumindest eines biobasierten endständigen Olefins, wobei das Olefin eine Kohlenstoffanzahl von 6 bis 45 aufweist,
b) Hinzugeben zumindest eines nicht modifizierten Siloxanpolymers,
c) Hinzugeben zumindest eines Übergangsmetall-Katalysators, welches optional in einem Lösemittel gelöst ist,
d) Umsetzung des Gemisches unter Rühren bei einer Temperatur von 65 °C bis 130 °C, wodurch sich das alkyl-modifizierte Silikonpolymers bildet und das erhaltene alkyl-modifizierte Silikonpolymers mindestens einen biobasierten Anteil von mindestens 45 Gew.-% bezogen auf die Masse des Polymers aufweist.

2. Verfahren gemäß Anspruch 1 **dadurch gekennzeichnet, dass** das zumindest eine nicht modifizierte Siloxanpolymer mindestens eine Einheit der Form - [Si(CH₃)HO]ₙ- mit n= 1 bis 100 umfasst.

3. Verfahren gemäß Anspruch 2 **dadurch gekennzeichnet, dass** das zumindest eine Siloxanpolymer eine weitere Einheit der Form -[Si(CH₃)₂O]ₘ- mit m = 1 bis 300 umfasst.

4. Verfahren gemäß mindestens einem der Ansprüche 1 bis 3 **dadurch gekennzeichnet, dass** das zumindest eine biobasierte endständige Olefin eine Kohlenstoffanzahl von 8 bis 30, meist bevorzugt 10 bis 20 aufweist.

5. Verfahren gemäß mindestens einem der Ansprüche 1 bis 4 **dadurch gekennzeichnet, dass** das zumindest eine biobasierte endständige Olefin zumindest eine Ester-, eine Carbonsäure- und/oder eine Hydroxyl-Gruppe aufweist.

6. Verfahren gemäß mindestens einem der Ansprüche 1 bis 5 **dadurch gekennzeichnet, dass** das zumindest eine biobasierte endständige Olefin keine weiteren zusätzlichen funktionellen Gruppen aufweist.

7. Verfahren gemäß mindestens einem der Ansprüche 1 bis 6 **dadurch gekennzeichnet, dass** das erhaltene alkyl-modifizierte Silikonpolymers mindestens einen biobasierten Anteil von 60 Gew.-% bis 98 Gew.-%, bevorzugt 80 Gew.-% bis 95 Gew.-% bezogen auf die Masse des Polymers aufweist.

8. Verfahren gemäß mindestens einem der Ansprüche 1 bis 7 **dadurch gekennzeichnet, dass** das zumindest eine biobasierte endständige Olefin aus einer natürlichen Quelle für Fettalkohole gewonnen wurde, insbesondere aus Fettalkoholen mit einer Kettenlänge von C = 6 bis 45.

9. Verwendung eines alkyl-modifizierten Silikonpolymers insbesondere erhalten durch ein Verfahren gemäß mindestens einem der Ansprüche 1 bis 7 als Hilfsstoff in kosmetischen und/oder Hygieneprodukten.
